# EUROPEAN PATENT APPLICATION

(11) **EP 4 778 454 A1**
(43) Date of publication of application: **22.07.2026**
(21) Application number: 26151342.8
(22) Date of filing: 12.01.2026
(51) Int. Cl.: A61B 5/024, A61B 5/00

(54) **MODES FOR HEART RATE MONITOR**

(30) Priority: 15.01.2025 US 202519022398
(71) Applicant: Meta Platforms Technologies, LLC, Menlo Park, CA 94025 (US)
(72) Inventor: HEMKUMAR, Aashna, Menlo Park, 94025 (US); SHAGA, Ravi Krishna, Menlo Park, 94025 (US)
(74) Representative: Murgitroyd & Company

(57) **Abstract**

A wearable device includes a light source, a light sensor, and processing logic. The light source is configured to emit illumination light. The light sensor is configured to generate light measurements of returning light. The returning light is a portion of the illumination light. The processing logic is configured to adjust a noise floor of the light measurements under certain lighting conditions.

## Description

### TECHNICAL FIELD

This disclosure relates generally to wearables, and in particular to heart rate monitoring.

### BACKGROUND INFORMATION

Heart monitor sensors are included in wearables, such as smartwatches and fitness trackers. Some heart rate sensors utilize photoplethysmography (PPG) technology to measure heart rate and rhythm. PPG sensors emit light through the skin and detect changes in blood flow, allowing for heart rate monitoring. These sensors can provide valuable insights into cardiovascular health and allow users to track their physical activity, stress levels, and overall well-being.

### SUMMARY

According to an aspect, there is provided a wearable device comprising:
a light source configured to emit illumination light;
a light sensor configured to generate light measurements of returning light, wherein the returning light is a portion of the illumination light; and
processing logic configured to:
   receive an ambient light measurement; and
   enter a low-noise mode for heart rate measurements when the light measurements are below a light threshold and the ambient light measurement is below an ambient threshold.

In one embodiment, the low-noise mode for heart rate measurements includes reducing a dark noise floor of measurements generated by the light sensor.

In one embodiment, the wearable device further comprises:
an ambient light cancellation circuit electrically coupled between the light sensor and the processing logic, wherein reducing the dark noise floor includes bypassing the ambient light cancellation circuit to measure the light measurements.

In one embodiment, the dark noise floor is below 20 pA.

In one embodiment, the dark noise floor is reduced by between 4x and 8x.

In one embodiment, the light threshold is 2 µA or lower current from the light sensor generated by the light measurements.

In one embodiment, the heart rate measurements are from photoplethysmography, PPG, analysis.

According to another aspect, there is provided a method comprising:
generating light measurements with a light sensor of a wearable;
receiving an ambient light measurement; and
adjusting a dark noise floor for heart rate measurements when the light measurements are below a light threshold and the ambient light measurement is below an ambient threshold.

In one embodiment, adjusting the dark noise floor for heart rate measurements includes adjusting the dark noise floor downward of the light measurements generated by the light sensor.

In one embodiment, adjusting the dark noise floor downward includes bypassing an ambient light cancellation circuit.

In one embodiment, the dark noise floor is adjusted downward by between 4x and 8x.

In one embodiment, the dark noise floor is below 20 pA.

In one embodiment, the light threshold is 2 µA or lower current from the light sensor generated by the light measurements.

In one embodiment, the heart rate measurements are from photoplethysmography, PPG, analysis.

According to a further aspect, there is provided a wearable device comprising:
a light source configured to emit illumination light;
a light sensor configured to generate light measurements of returning light,
wherein the returning light is a portion of the illumination light; a memory including a skin tone classifier value; and
processing logic communicatively coupled to the memory, wherein the processing logic is configured to:
   receive an ambient light measurement; and
   enter a low-noise mode for heart rate measurements when the skin tone classifier value is at a pre-determined value and the ambient light measurement is below an ambient threshold.

In one embodiment, the low-noise mode for heart rate measurements includes reducing a dark noise floor of measurements generated by the light sensor.

In one embodiment, the wearable device further comprises:
an ambient light cancellation circuit electrically coupled between the light sensor and the processing logic, wherein reducing the dark noise floor includes bypassing the ambient light cancellation circuit to measure the light measurements.

In one embodiment, the dark noise floor is reduced by between 4x and 8x.

In one embodiment, the dark noise floor is below 20 pA.

In one embodiment, the heart rate measurements are from photoplethysmography, PPG, analysis.

It will be appreciated that any features described herein as being suitable for incorporation into one or more aspects or embodiments of the present disclosure are intended to be generalizable across any and all aspects and embodiments of the present disclosure. Other aspects of the present disclosure can be understood by those skilled in the art in light of the description, the claims, and the drawings of the present disclosure. The foregoing general description and the following detailed description are exemplary and explanatory only and are not restrictive of the claims.

### BRIEF DESCRIPTION OF THE DRAWINGS

Non-limiting and non-exhaustive embodiments of the invention are described with reference to the following figures, wherein like reference numerals refer to like parts throughout the various views unless otherwise specified.
FIG. 1 illustrates a heart rate monitoring system that includes a wearable including a heart rate monitoring system measuring the blood flow in tissue, in accordance with aspects of the disclosure.
FIG. 2 illustrates an example wearable that may include a heart rate monitoring system, in accordance with aspects of the disclosure.
FIG. 3 illustrates an example circuit block diagram for a transmit path for driving a light source to emit illumination light, in accordance with aspects of the disclosure.
FIG. 4 illustrates an example circuit block diagram of a receive path for generating light measurements and ambient light measurements, in accordance with aspects of the disclosure.
FIG. 5 illustrates a flow chart for an example process of adjusting a noise floor for heart rate measurements, in accordance with aspects of the disclosure.

### DETAILED DESCRIPTION

Embodiments of modes for monitoring heart rates are described herein. In the following description, numerous specific details are set forth to provide a thorough understanding of the embodiments. One skilled in the relevant art will recognize, however, that the techniques described herein can be practiced without one or more of the specific details, or with other methods, components, materials, etc. In other instances, well-known structures, materials, or operations are not shown or described in detail to avoid obscuring certain aspects.

Reference throughout this specification to "one embodiment" or "an embodiment" means that a particular feature, structure, or characteristic described in connection with the embodiment is included in at least one embodiment of the present invention. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

In aspects of this disclosure, visible light may be defined as having a wavelength range of approximately 380 nm - 700 nm. Non-visible light may be defined as light having wavelengths that are outside the visible light range, such as ultraviolet light and infrared light. Infrared light having a wavelength range of approximately 700 nm - 1 mm includes near-infrared light. In aspects of this disclosure, near-infrared light may be defined as having a wavelength range of approximately 700 nm - 1.6 µm.

In aspects of this disclosure, the term "transparent" may be defined as having greater than 90% transmission of light. In some aspects, the term "transparent" may be defined as a material having greater than 90% transmission of visible light.

PPG heart rate sensing techniques are generally quite accurate. However, there are contexts where PPG heart rate sensing techniques have sub-optimal performance. One particular context where PPG heart rate sensing techniques may have sub-optimal performance is when a wearable having a PPG sensing system is worn by an individual with darker skin tones, having lower perfusion. This is because the return photodiode current of a PPG signal is more highly attenuated for users with higher melanin concentrations corresponding to low perfusion tissue. As such, the return photodiode signal may have low direct current (DC) and alternating current (AC) signals. Existing technologies attempt to compensate for this by overdriving the PPG sensing by maximizing the drive current of the light source (e.g. LED) and increasing the integration time and sampling rate. However, this results in significant power consumption and reduced battery life for some users.

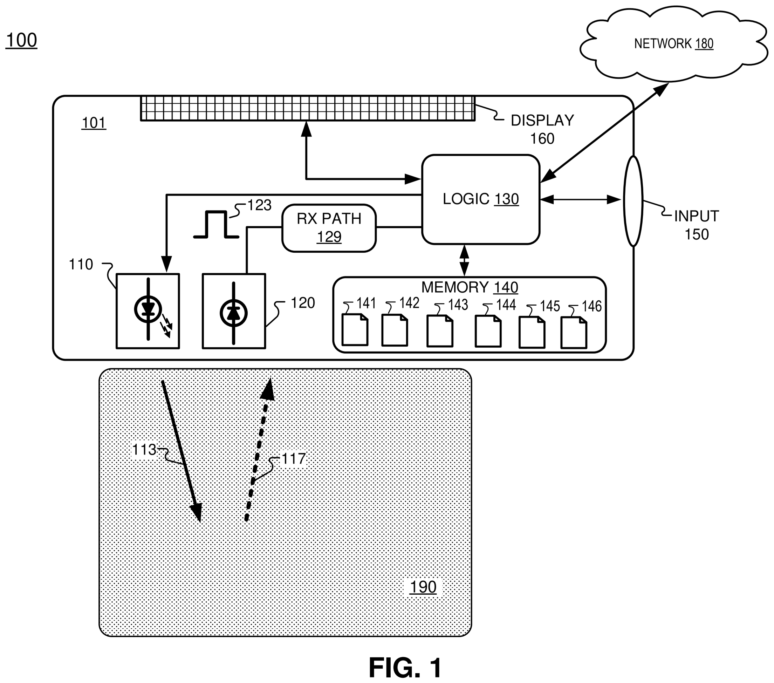

In implementations of the disclosure, a dark noise floor for heart rate measurements is adjusted when light measurements are below a light threshold (which may indicate a likelihood of a user with low perfusion tissue) and an ambient light measurement is below an ambient threshold. Adjusting the dark noise floor for a light measurements in a heart rate monitoring context may significantly increase the signal-to-noise ratio (SNR) of the light measurements without needing to overdrive the PPG sensing system. These and other embodiments are described in more detail in connection with FIGs. 1-5.

FIG. 1 illustrates a heart rate monitoring system 100 that includes a wearable 101 including a heart rate monitoring system measuring the blood flow in tissue 190, in accordance with aspects of the disclosure. Heart rate monitoring system 100 may be a PPG heart rate sensing system.

The example wearable 101 illustrated in FIG. 1 includes input(s) 150. Input 150 may include buttons, dials, and/or touch-sensitive sensors, for example. Input(s) 150 are communicatively coupled to processing logic 130, in FIG. 1. In some implementations, inputs for wearable 101 are received via a touch-screen overlaying display 160. Display 160 may be a liquid crystal display (LCD) or an organic light-emitting-diode (OLED) display, for example. Display 160 is also communicatively coupled to processing logic 130, in FIG. 1.

In operation, light source 110 emits illumination light 113 into tissue 190. Light source 110 may include an LED or a laser diode, for example. Light source 110 may emit visible illumination light 113. Illumination light 113 may be green visible light. Illumination light 113 may be red visible light. Light source 110 may be an infrared light source emitting infrared illumination light 113. Light source 110 may be a near-infrared light source emitting near-infrared illumination light 113. For example, light source 110 may be centered around 850 nm or 940 nm.

Illumination light propagates into tissue 190 that includes blood vessels and blood capillaries. A portion of illumination light 113 is reflected back through tissue and exits tissue 190 as returning light 117 that is measured by light sensor 120. Light sensor 120 may include a photodiode, as illustrated in FIG. 1. In some implementations, light sensor 120 includes an optical filter tuned to receive the wavelength of illumination light 113 (and returning light 117) while blocking out other light wavelengths. In other words, the filter on light sensor 120 may be matched to light source 110.

Light sensor 120 generates signals 123 in response to incident light. When light source is not activated (not emitting illumination light 113), light sensor 120 may generate ambient light measurements as signal 123 to measure the contribution of light from the external environment. When light source 110 is activated (emitting illumination light 113), light sensor 120 generates light measurements of returning light 117 as signal 123. Processing logic 130 may be configured to coordinate driving light source 110 and the sampling of signals 123 by light sensor 120. By generating many light measurements, the heart rate of a user of wearable 101 can be determined due to bloodflow changes in tissue 190 and the corresponding absorption of illumination light 113 in the blood. For example, more blood in tissue 190 will absorb more of illumination light 113 and thus returning light 117 will be of a decreased intensity when there is more blood in tissue 190 while returning light 117 will have increased intensity when there is less blood present in tissue 190. Patterns can then be extracted from the many light measurements and the patterns can be analyzed for heart rate monitoring.

Processing logic 130 may receive signals 123 from light sensor 120 and store the many signals as light measurements in memory 140. Receive path logic 129 may be coupled between light sensor 120 and processing logic 130. Receive path logic 129 may include analog and/or digital circuitry to amplify and/or condition signal 123 for input into processing logic 130. Processing logic 130 may process and analyze light measurements stored in memory 140 to determine heart rate, sleep patterns, fitness data, (or otherwise) and then display results to the user via display 160, in some implementations.

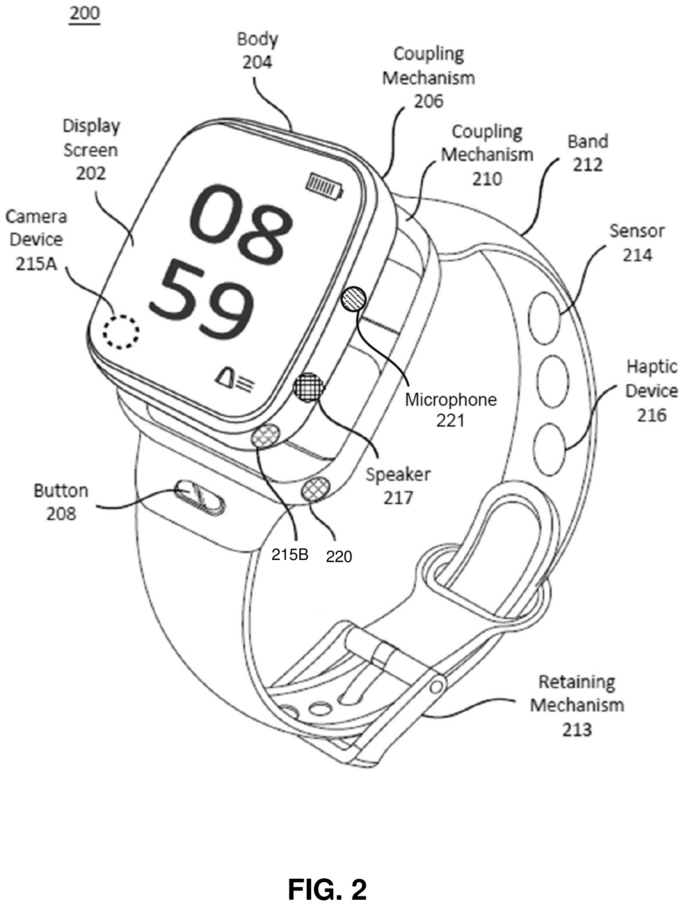

FIG. 2 illustrates an example wearable 200 that may include heart rate monitoring systems such as system 100 of FIG. 1, in accordance with aspects of the disclosure. The wearable illustrated in FIG. 2 is just an example form-factor and the embodiments of the disclosure may be used in various form factors. In some embodiments, a user may select a function by interacting with the button 208 (e.g., by pushing, turning, etc.). In some embodiments, a user may select a function by interacting with the display screen 202. For example, the display screen 202 is a touchscreen and the user may select a particular function by touching the display screen 202, in some implementations. The functions executed by wearable 200 may include, without limitation, displaying visual content to the user (e.g., displaying visual content on the display screen 202), presenting audio content to the user (e.g., presenting audio content via the speaker 217), sensing user input (e.g., sensing a touch of button 208, sensing biometric data with the one or more sensors 214, sensing neuromuscular signals with the one or more sensors 214, etc.), capturing audio content (e.g., capturing audio with microphone 221), capturing data describing a local area (e.g., with a front-facing camera device 215A and/or a rear-facing camera device 215B), communicating wirelessly (e.g., via cellular, near field, Wi-Fi, personal area network, etc.), communicating via wire (e.g., via the port), determining location (e.g., sensing position data with a sensor 214), determining a change in position (e.g., sensing change(s) in position with an IMU), determining an orientation and/or acceleration (e.g., sensing orientation and/or acceleration data with an IMU), providing haptic feedback (e.g., with the haptic device 216), etc.

The display screen 202 may display visual content to the user. The displayed visual content may be oriented to the eye gaze of the user such that the content is easily viewed by the user. Traditional displays on smartwatches may orient the visual content in a static manner such that when a user moves or rotates the smartwatch, the content may remain in the same position relative to the smartwatch causing difficulty for the user to view the content. Embodiments of the present disclosure may orient (e.g., rotate, flip, stretch, etc.) the displayed content such that the displayed content remains in substantially the same orientation relative to the eye gaze of the user (e.g., the direction in which the user is looking). The displayed visual content may also be modified based on the eye gaze of the user. For example, in order to reduce the power consumption of wearable 200, the display screen 202 may dim the brightness of the displayed content, pause the displaying of video content, or power down the display screen 202 when it is determined that the user is not looking at the display screen 202. In some examples, one or more sensors 214 of the wearable 200 may determine an orientation of the display screen 202 relative to an eye gaze direction of the user

Wearable 200 may be considered a smartwatch. FIG. 2 illustrates a coupling mechanism 206, a camera device 215A, a display screen 202, a button 108, a speaker 217, a microphone 221, and a release mechanism 220 associated with the watch body 204. FIG. 2 illustrates a coupling mechanism 210, a retaining mechanism 213, the sensor 214, the haptic device 216, and a release mechanism 220 associated with the watch band 212. In some implementations, heart rate sensing components (e.g. light source 110 and light sensor 120) are disposed on an underside of the watch body 204. The heart rate sensing components may also be included in band 212, such as within sensor 214, in some implementations.

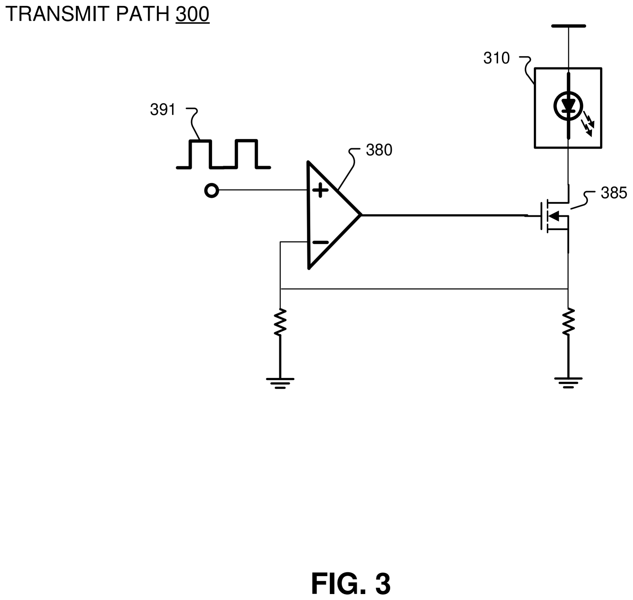

FIG. 3 illustrates an example circuit block diagram for a transmit path 300 for driving a light source 310 to emit illumination light, in accordance with aspects of the disclosure. Transmit path 300 includes an amplifier 380 (e.g. an op-amp) driving a n-channel field-effect transistor (nFET) 385 to control a current through light source 310. Light source 310 may include an LED or a laser diode, for example. Light source 310 may emit visible illumination light. The illumination light 113 be green visible light. The illumination light may be red visible light. Light source 310 may be an infrared light source emitting infrared illumination light. Light source 310 may be a near-infrared light source emitting near-infrared illumination light. For example, light source 310 may be centered around 850 nm or 940 nm.

In FIG. 3, amplifier 380 receives driving signal 391. Driving signal 391 controls the voltage output of amplifier 380, which modulates the voltage on the gate of nFET 385 to modulate the current through nFET 385, and consequently, light source 310. Driving signal 391 may be driven onto amplifier 380, by processing logic 130, in some implementations. The driving signal 391 may turn light source 310 on and off as well as modulate the intensity of the illumination light emitted from light source 310. Notably, in some implementations, more than one light source is included in transmit path 300 and each light source may be modulated independently. In these implementations, the transmit path 300 of FIG. 3 may be duplicated and processing logic 130 may control the additional transmit path(s) with separate driving signals.

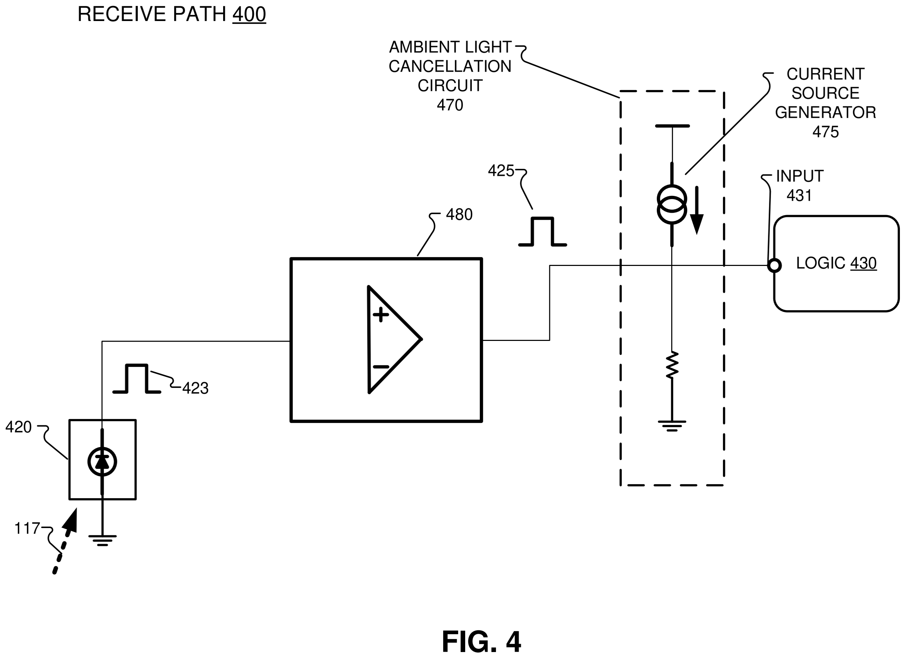

FIG. 4 illustrates an example circuit block diagram of receive path 400 for generating light measurements and ambient light measurements, in accordance with aspects of the disclosure. Receive path 400 includes light sensor 420, amplifier stage 480, ambient light cancellation circuit 470 and processing logic 430. Receive path 400 may be considered an analog front end (AFE) for processing logic 430. Receive path 129 may use aspects of receive path 400.

Light sensor 420 may include a photodiode. In some implementations, light sensor 420 includes an optical filter tuned to receive the wavelength of returning light 117 while blocking out other light wavelengths.

In operation, light sensor 420 generates signal 423 in response to returning light 117 incident on light sensor 420. Signal 423 may be a current signal from a photodiode, in some implementations. Optional amplifier stage 480 may generate amplified signal 425 in response to receiving signal 423. Amplifier stage 480 may include one or more op-amps or transistors to amplify signal 423.

Ambient light cancellation circuit 470 is coupled between light sensor 420 and processing logic 430. Ambient light cancellation circuit 470 is configured to cancel out the contribution of ambient light that has been included in signal 423/425. The illustrated ambient light cancellation circuit 470 includes a current source generator 475 to generate a cancelling current that cancels out the contribution of ambient light included in signal 423/425. In some implementations, current source generator 475 is driven by a digital-to-analog converter (DAC) controlled by processing logic 430. The DAC may be included in the same chip as processing logic 430 or be external to processing logic 430. Hence, the current source generator 475 may be controlled to adjust signal 423/425 received by input 431 of processing logic 430, according to an ambient light measurement. The ambient light measurement may be generated by light sensor 420 when the light source 110/310 is not activated (not emitting illumination light) or the ambient light measurement may be made by a separate ambient light sensor. Input 431 of processing logic 430 may be an analog-to-digital converter (ADC) that is internal to processing logic 430 or external to processing logic 430. The features of processing logic 430 may be included in processing logic 130.

Referring again to FIG. 1, processing logic 130 may be configured to receive an ambient light measurement. The ambient light measurement may be generated by light sensor 120 or by a separate light sensor (not illustrated). Processing logic 130 may store the ambient light measurement to memory 140 as ambient light measurement 141. Processing logic 130 may also generate light measurements with light sensor 120. Generating the light measurements may include processing logic 130 driving light source to emit illumination light 113 while light sensor 120 measures returning light 117. Processing logic 130 may store the light measurements to memory 140 as light measurements 142.

To assist in measuring heart rate for different skin tones (low perfusion skin tones in particular) the noise floor for light measurements may be adjusted. Light measurements below a light threshold value may indicate a user with darker skin (e.g. FP5 or FP6 on the Fitzpatrick scale). In an implementation, when (1) the light measurements 142 are below a light threshold 143; and (2) the ambient light measurement 141 is below an ambient light threshold 144, processing logic 130 may be configured to enter a low-noise mode for heart rate measurements. In an implementation, the low-noise mode for heart rate measurements includes reducing a dark noise floor of the measurements generated by the light sensor. In an implementation, reducing the dark noise floor includes bypassing an ambient light cancellation circuit (e.g. ambient light cancellation circuit 470) to measure the light measurements. Bypassing the ambient light cancellation circuit may include pausing driving the current source generator 475 (e.g. not driving the DAC to generate the cancelling current) included in ambient light cancellation circuit 470.

Bypassing the ambient light cancellation circuit improves the SNR for signals 423/425 because operating current source generator 475 may be the largest noise contributor in the receive path. However, to bypass the ambient light cancellation circuit, the ambient light measurement must be below the ambient light threshold. Otherwise, the ambient light measured by light sensor 120 dominates signal 123 and heart rate measurements become unreliable.

In an implementation, light threshold 143 corresponds to signal 123 being 2 µA or lower current during the light measurements of returning light 117. In some implementations, the dark noise floor in the low-noise mode is reduced between 4X and 8X. In some implementations, the dark noise floor is reduced to below 20 pA.

In some implementations, memory 140 includes a skin tone classifier value 145. The skin tone classifier value 145 may be based on previous light measurements by wearable 101. The skin tone classifier value 145 may be inputted by the user as a setting of wearable 101. The skin tone classifier value 145 may be generated from a photograph of the user that is stored in memory 140 or that processing logic 130 may access via network 180. Processing logic 130 may receive an ambient light measurement (e.g. ambient light measurement 141). When (1) skin tone classifier value 145 is at a pre-determined value 146; and (2) the ambient light measurement 141 is below an ambient light threshold 144, processing logic 130 may be configured to enter a low-noise mode for heart rate measurements. The pre-determined value 146 stored in memory 140 may correspond with skin tone types on the Fitzpatrick scale, in some implementations.

In implementations utilizing skin tone classifier value 145, the low-noise mode for heart rate measurements may include reducing a dark noise floor of measurements generated by the light sensor. In an implementation, reducing the dark noise floor includes bypassing an ambient light cancellation circuit (e.g. ambient light cancellation circuit 470) to measure the light measurements for heart rate monitoring. In some implementations, the dark noise floor in the low-noise mode is reduced between 4X and 8X. In some implementations, the dark noise floor is reduced to below 20 pA.

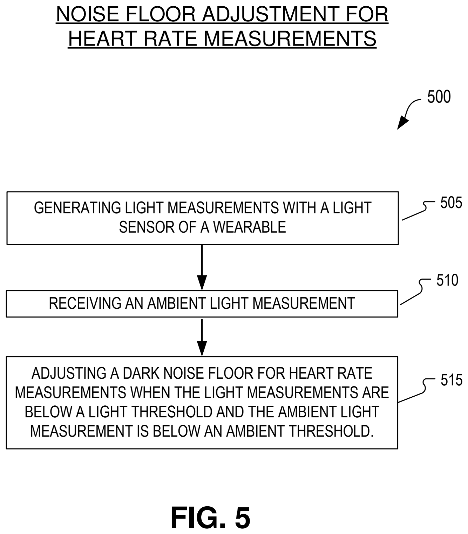

FIG. 5 illustrates a flow chart for an example process 500 of adjusting a noise floor for heart rate measurements, in accordance with aspects of the disclosure. The order in which some or all of the process blocks appear in process 500 should not be deemed limiting. Rather, one of ordinary skill in the art having the benefit of the present disclosure will understand that some of the process blocks may be executed in a variety of orders not illustrated, or even in parallel.

In process block 505, light measurements are generated with a light sensor of a wearable.

In process block 510, an ambient light measurement is received.

In process block 515, a dark noise floor for heart rate measurements is adjusted when the light measurements are below a light threshold and the ambient light measurement is below an ambient threshold.

In an implementation of process 500, adjusting the dark noise-floor for heart rate measurements includes adjusting the dark noise floor downward of the light measurements generated by the light sensor. Adjusting the dark noise floor downward may include bypassing an ambient light cancellation circuit (e.g. circuit 470).

In an implementation, the dark noise floor is adjusted downward by between 4x and 8x. The dark noise floor may be below 20 pA, in some implementations.

Embodiments of the invention may include or be implemented in conjunction with an artificial reality system. Artificial reality is a form of reality that has been adjusted in some manner before presentation to a user, which may include, e.g., a virtual reality (VR), an augmented reality (AR), a mixed reality (MR), a hybrid reality, or some combination and/or derivatives thereof. Artificial reality content may include completely generated content or generated content combined with captured (e.g., real-world) content. The artificial reality content may include video, audio, haptic feedback, or some combination thereof, and any of which may be presented in a single channel or in multiple channels (such as stereo video that produces a three-dimensional effect to the viewer). Additionally, in some embodiments, artificial reality may also be associated with applications, products, accessories, services, or some combination thereof, that are used to, e.g., create content in an artificial reality and/or are otherwise used in (e.g., perform activities in) an artificial reality. The artificial reality system that provides the artificial reality content may be implemented on various platforms, including a head-mounted display (HMD) connected to a host computer system, a standalone HMD, a mobile device or computing system, or any other hardware platform capable of providing artificial reality content to one or more viewers.

The term "processing logic" (e.g. processing logic 130 or 430) in this disclosure may include one or more processors, microprocessors, multi-core processors, Application-specific integrated circuits (ASIC), and/or Field Programmable Gate Arrays (FPGAs) to execute operations disclosed herein. In some embodiments, memories (not illustrated) are integrated into the processing logic to store instructions to execute operations and/or store data. Processing logic may also include analog or digital circuitry to perform the operations in accordance with embodiments of the disclosure.

A "memory" or "memories" (e.g. memory 140) described in this disclosure may include one or more volatile or non-volatile memory architectures. The "memory" or "memories" may be removable and non-removable media implemented in any method or technology for storage of information such as computer-readable instructions, data structures, program modules, or other data. Example memory technologies may include RAM, ROM, EEPROM, flash memory, CD-ROM, digital versatile disks (DVD), high-definition multimedia/data storage disks, or other optical storage, magnetic cassettes, magnetic tape, magnetic disk storage or other magnetic storage devices, or any other non-transmission medium that can be used to store information for access by a computing device.

Networks may include any network or network system such as, but not limited to, the following: a peer-to-peer network; a Local Area Network (LAN); a Wide Area Network (WAN); a public network, such as the Internet; a private network; a cellular network; a wireless network; a wired network; a wireless and wired combination network; and a satellite network.

Communication channels may include or be routed through one or more wired or wireless communication utilizing IEEE 802.11 protocols, short-range wireless protocols, SPI (Serial Peripheral Interface), I²C (Inter-Integrated Circuit), USB (Universal Serial Port), CAN (Controller Area Network), cellular data protocols (e.g. 3G, 4G, LTE, 5G), optical communication networks, Internet Service Providers (ISPs), a peer-to-peer network, a Local Area Network (LAN), a Wide Area Network (WAN), a public network (e.g. "the Internet"), a private network, a satellite network, or otherwise.

A computing device may include a desktop computer, a laptop computer, a tablet, a phablet, a smartphone, a feature phone, a server computer, or otherwise. A server computer may be located remotely in a data center or be stored locally.

The processes explained above are described in terms of computer software and hardware. The techniques described may constitute machine-executable instructions embodied within a tangible or non-transitory machine (e.g., computer) readable storage medium, that when executed by a machine will cause the machine to perform the operations described. Additionally, the processes may be embodied within hardware, such as an application specific integrated circuit ("ASIC") or otherwise.

A tangible non-transitory machine-readable storage medium includes any mechanism that provides (i.e., stores) information in a form accessible by a machine (e.g., a computer, network device, personal digital assistant, manufacturing tool, any device with a set of one or more processors, etc.). For example, a machine-readable storage medium includes recordable/non-recordable media (e.g., read only memory (ROM), random access memory (RAM), magnetic disk storage media, optical storage media, flash memory devices, etc.).

The above description of illustrated embodiments of the invention, including what is described in the Abstract, is not intended to be exhaustive or to limit the invention to the precise forms disclosed. While specific embodiments of, and examples for, the invention are described herein for illustrative purposes, various modifications are possible within the scope of the invention, as those skilled in the relevant art will recognize.

These modifications can be made to the invention in light of the above detailed description. The terms used in the following claims should not be construed to limit the invention to the specific embodiments disclosed in the specification. Rather, the scope of the invention is to be determined entirely by the following claims, which are to be construed in accordance with established doctrines of claim interpretation.

## Claims

1. A wearable device comprising:
a light source configured to emit illumination light;
a light sensor configured to generate light measurements of returning light, wherein the returning light is a portion of the illumination light; and
processing logic configured to:
receive an ambient light measurement; and
enter a low-noise mode for heart rate measurements when the light measurements are below a light threshold and the ambient light measurement is below an ambient threshold.

2. The wearable device of claim 1, wherein the low-noise mode for heart rate measurements includes reducing a dark noise floor of measurements generated by the light sensor.

3. The wearable device of claim 2 further comprising:
an ambient light cancellation circuit electrically coupled between the light sensor and the processing logic, wherein reducing the dark noise floor includes bypassing the ambient light cancellation circuit to measure the light measurements.

4. The wearable device of claim 2 or claim 3, wherein:
the dark noise floor is below 20 pA, and/or
the dark noise floor is reduced by between 4x and 8x, and/or
the light threshold is 2 µA or lower current from the light sensor generated by the light measurements.

5. The wearable device of any preceding claim, wherein the heart rate measurements are from photoplethysmography, PPG, analysis.

6. A method comprising:
generating light measurements with a light sensor of a wearable;
receiving an ambient light measurement; and
adjusting a dark noise floor for heart rate measurements when the light measurements are below a light threshold and the ambient light measurement is below an ambient threshold.

7. The method of claim 6, wherein adjusting the dark noise floor for heart rate measurements includes adjusting the dark noise floor downward of the light measurements generated by the light sensor.

8. The method of claim 7, wherein adjusting the dark noise floor downward includes bypassing an ambient light cancellation circuit.

9. The method of claim 7 or claim 8, wherein the dark noise floor is adjusted downward by between 4x and 8x.

10. The method of any of claims 7 to 9, wherein:
the dark noise floor is below 20 pA, and/or
the light threshold is 2 µA or lower current from the light sensor generated by the light measurements.

11. The method of any of claims 6 to 10, wherein the heart rate measurements are from photoplethysmography, PPG, analysis.

12. A wearable device comprising:
a light source configured to emit illumination light;
a light sensor configured to generate light measurements of returning light,
wherein the returning light is a portion of the illumination light;
a memory including a skin tone classifier value; and
processing logic communicatively coupled to the memory, wherein the processing logic is configured to:
receive an ambient light measurement; and
enter a low-noise mode for heart rate measurements when the skin tone classifier value is at a pre-determined value and the ambient light measurement is below an ambient threshold.

13. The wearable device of claim 12, wherein the low-noise mode for heart rate measurements includes reducing a dark noise floor of measurements generated by the light sensor,
and optionally further comprising:
an ambient light cancellation circuit electrically coupled between the light sensor and the processing logic, wherein reducing the dark noise floor includes bypassing the ambient light cancellation circuit to measure the light measurements.

14. The wearable device of claim 13, wherein:
the dark noise floor is reduced by between 4x and 8x, and/or
the dark noise floor is below 20 pA.

15. The wearable device of any of claims 12 to 14, wherein the heart rate measurements are from photoplethysmography, PPG, analysis.
